# EUROPÄISCHE PATENTSCHRIFT

(11) **EP 3 067 091 B1**
(45) Veröffentlichungstag und Bekanntmachung des Hinweises auf die Patenterteilung: **29.07.2020**
(21) Anmeldenummer: 16155474.6
(22) Anmeldetag: 12.02.2016
(51) Int. Cl.: A61N 1/39, A61B 5/0402, A61N 1/362

(54) **DISLOKATIONSSENSOR**
DISLOCATION SENSOR
CAPTEUR DE DISLOCATION

(30) Priorität: 13.03.2015 US 201562132514 P
(43) Veröffentlichungstag der Anmeldung: 14.09.2016
(73) Patentinhaber: BIOTRONIK SE & Co. KG, 12359 Berlin (DE)
(72) Erfinder: Dörr, Thomas, 12437 Berlin (DE); Stürmer, Uwe, 10119 Berlin (DE)
(74) Vertreter: Galander, Marcus

(56) Entgegenhaltungen:
- WO-A1-96/05768
- US-A1- 2003 069 608
- US-A1- 2006 047 333
- US-A1- 2006 116 747
- US-A1- 2010 114 217
- US-A1- 2013 345 536
- US-A1- 2014 163 639

## Beschreibung

Die Erfindung betrifft ein aktives implantierbares medizinisches Therapie- und/oder Monitoringsystem, das mindestens ein Implantat umfasst.

Beispiele für derartige aktive implantierbare medizinische Therapie- und/oder Monitoringsysteme sind implantierbare Herz-Therapie- und/oder Herz-Monitoring-Geräte, beispielsweise Herzschrittmacher oder Kardioverter/Defibrillatoren. Herzschrittmacher sind häufig Therapiesysteme, die einen implantierbaren Pulsgenerator (den eigentlichen Herzschrittmacher) und daran angeschlossene Elektrodenleitungen mit Elektrodenpolen aufweisen.

Typischerweise ist für eine jeweilige zu stimulierende Herzkammer (z.B. rechter oder linker Ventrikel oder rechtes oder linkes Atrium) jeweils eine eigene Elektrodenleitung vorgesehen.

Über einen oder mehrere Stimulationselektrodenpole einer jeweiligen Elektrodenleitung kann ein Herzschrittmacher einen elektrischen Stimulationsimpuls an das Muskelgewebe einer jeweiligen Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet.

Daneben dienen die Elektrodenleitungen mit ihren Elektrodenpolen auch zum Erfassen von Kontraktionen einer jeweiligen Herzkammer. Das Erfassen natürlicher Ereignisse dient bei bekannten Demand-Schrittmachern, beispielsweise der Unterdrückung (Inhibierung) der Abgabe von Stimulationsimpulsen an eine entsprechende Herzkammer, falls das natürliche Ereignis in einem Zeitfenster vor der geplanten Abgabe eines Stimulationsimpulses an diese Herzkammer erfasst wird.

In einem Elektrokardiogramm sind mit einer Kontraktion des Ventrikels einhergehende, eine Depolarisation der Herzmuskelzellen widerspiegelnde Aktionspotenziale als sogenannte Q-Zacke zu erkennen, während sich die mit der Entspannung des Myokards einhergehende Repolarisierung der Herzmuskelzellen in einer sogenannten T-Welle widerspiegelt.

Beim gesunden Menschen wird der jeweilige Herzrhythmus durch den vom autonomen Nervensystem gesteuerten Sinusknoten bestimmt. Dieser erregt per Reizleitung das rechte Atrium eines menschlichen Herzens und weiter über den AV-Knoten den (rechten) Ventrikel des Herzens. Ein vom Sinusknoten ausgehender natürlicher Herzrhythmus wird daher auch als Sinusrhythmus bezeichnet und führt zu jeweils natürlichen Kontraktionen der jeweiligen Herzkammer, die als natürliche (intrinsische) Ereignisse erfasst werden können.

Das Erfassen solcher natürlicher (intrinsischer) Ereignisse erfolgt durch Ableiten der elektrischen Potenziale des Myokards der jeweiligen Herzkammer mit Hilfe von Wahrnehmungselektroden, die Teil einer entsprechenden Elektrodenleitung sind. Dabei können die Wahrnehmungselektrodenpole gleichzeitig die Stimulationselektrodenpole sein und abwechselnd als Stimulations- und als Wahrnehmungselektrodenpol verwendet werden. Typischerweise ist für das Sensing - d.h. die Wahrnehmung intrinsischer Ereignisse - ein Wahrnehmungselektrodenpolpaar vorgesehen, das von zwei benachbarten Elektrodenpolen, nämlich einer Spitzenelektrode (Tip-Elektrode) und einer Ringelektrode, gebildet ist, von denen die Spitzenelektrode auch als Stimulationselektrodenpol dient. Auf diese Weise erfolgt eine bipolare Ableitung eines intrakardialen Elektrokardiogramms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischerweise über den Coronarsinus und eine von diesem abzweigende Lateralvene in die Nähe des linken Ventrikels ragt und dort eine kleinflächige Stimulations- und/oder Wahrnehmungselektrode aufweisen kann.

In Bezug auf die hierin verwendeten Bezeichnungen sei darauf hingewiesen, dass im Rahmen dieses Textes mit den Begriffen Stimulations- oder Wahrnehmungselektrode ein jeweiliger Elektrodenpol an einer Elektrodenleitung gemeint ist, also derjenige Teil einer Elektrodenleitung, über den Stimulationsimpulse abgegeben oder elektrische Potenziale aufgenommen werden. Es sei auch darauf hingewiesen, dass es auch üblich ist, mit "Stimulationselektrode" eine der Stimulation dienende Elektrodenleitung zu bezeichnen.

Die Wahrnehmungselektrodenpole sind im Betrieb des Herzstimulators mit entsprechenden Wahrnehmungseinheiten verbunden, die ausgebildet sind, ein jeweiliges über einen Wahrnehmungselektrodenpol (bzw. ein Wahrnehmungselektrodenpolpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Durch Dislokation ("Verrutschen" oder "Verschieben") einer Elektrodenleitung samt ihrer Wahrnehmungselektrodenpole kann es dazu kommen, dass sich Amplitude und/oder Form der über eine oder mehrere Wahrnehmungselektrodenpole aufgenommene Signale ändert, ohne dass dies physiologisch bedingt ist. Dies stellt in Bezug auf eine zuverlässige Auswertung erfasster Signale ein Problem dar, so dass der Wunsch besteht, eine Dislokation einer Elektrodenleitung und/oder ihrer Elektrodenpole zu erkennen, zumal aus erfassten Signalen eine Reihe relevanter Therapieparameter abgeleitet werden.

In Bezug auf eine möglicherweise die Wahrnehmung von Ereignissen beinflussende Dislokation von Wahrnehmungselektrodenpolen sind Lösungsansätze bekannt, eine solche Dislokation zu erkennen. Bekannte Lösungsansätze zur Erkennung einer Dislokation einer Stimulationselektrode basieren auf der Auswertung der Wahrnehmungsamplitude, der Elektrodenimpedanzen und der Stimulationsreizschwellen.

In US 7,664,550 ist ein Verfahren beschrieben, die Dislokation einer linksventrikulären Elektrodenleitung mit ihren Elektrodenpolen an einer veränderten Signalamplitude, Morphologie oder einem geänderten zeitlichen Abstand zum atrialen Signal zu erkennen.

Bekannte Ansätze zum Erkennen von Elektrodenlagefehlern bedienen sich der Messung und Auswertung folgender Parameter:
- Signalamplituden von Biosignalen
- Impedanzen
- Reizschwellen
- Signalformanalysen (auch Vergleich in mehreren Kanälen).

Die US Patentanmeldung US 20140163639 A1 beschreibt ein System für die Neurostimulation für die Therapie an einem
Patienten, bestehend aus einer Elektrodenleitung zur Neurostimulation, die zur Implantation in ein Zielgewebe geeignet ist und einem Neurostimulator, der dafür ausgelegt ist, Signale an die Elektrodenleitung zur Neurostimulation abzugeben. Das System verfügt über eine Schaltung, die dafür vorgesehen ist Messungen vorzunehmen, um Hinweise für eine dreidimensionale Migration der Elektrodenleitung zu erhalten.

Der Erfindung liegt die Aufgabe zugrunde, eine unzulässige Lageänderung eines elektronischen Implantates und/oder einer implantierten Elektrodenleitung frühzeitig und zuverlässig erkennen zu können. Erfindungsgemäß wird diese Aufgabe durch ein aktives implantierbares medizinisches Therapie- und/oder Monitoringsystem gemäß Anspruch 1 gelöst, welches mindestens ein Implantat umfasst, in dem wenigstens ein Ultraschallwandler (Ultraschalltransducer) zum Abgeben und Aufnehmen von Ultraschallsignalen vorgesehen ist, der direkt oder indirekt mit einer Steuer- und Auswerteeinheit verbunden ist, die ausgebildet ist, ein Abgeben von Ultraschallsignalen durch den Ultraschallwandler zyklisch oder getriggert auszulösen und empfangene Ultraschallsignale so auszuwerten, dass die Steuer- und Auswerteeinheit eine jeweils aktuelle Lage oder Lageänderung des Implantates und/oder einer anderen Systemkomponente des aktiven implantierbaren medizinischen Therapie- und/oder Monitoringsystems, wie z.B. einer Elektrodenleitung, erkennt.

Ein derartiges System erlaubt es für alle elektronischen Implantate und Elektrodenleitungen eine ungewollte Lageänderung zuverlässiger und frühzeitig durch das Implantat selbst erkennen zu können.

Das System umfasst beispielsweise ein aktives elektronisches Implantat zur dauerhaften Implantation im menschlichen Körper, das direkt oder indirekt mit mindestens einem Ultraschallwandler (Transducer) und einer Steuer- und Auswerteeinheit verbunden ist, die mit dem Ultraschallwandler derart verbunden ist, dass diese zyklisch oder getriggert Ultraschallsignale aussendet und empfängt und die empfangenen Signale auswertet, um die implantierte Lage des Implantates und/oder der Elektrodenleitung zu bestätigen oder eine Dislokation zu erkennen.

Es ergibt sich somit beispielsweise eine Vorrichtung zur verbesserten Erkennung von Elektroden- oder Implantatlagefehlern, bei der mindestens ein Ultraschallwandler mit dem Implantatgehäuse oder der Elektrodenleitung derart verbunden ist, dass über eine Messung der Ultraschallreflektion (Amplituden) und/oder Signallaufzeiten und/oder Frequenzverschiebungen eine unzulässige Lageveränderung der Elektrodenleitung oder des Implantates erkannt wird.

Eine derartige Vorrichtung bietet den Vorteil, ungewollte Lageänderungen eines elektronischen Implantates oder einer implantierten Elektrodenleitung frühzeitig und zuverlässig erkennen zu können.

Die Erfindung schließt die Erkenntnis ein, dass bekannte Methoden zur Elektroden- oder Implantatlagefehlererkennung hinsichtlich ihrer Sensitivität und Spezifität aufgrund ihrer indirekten Messmethode eingeschränkt sind und auch nur bei implantierbaren Systemen angewendet werden können, bei denen die weiter oben aufgelisteten Parameter bauartbedingt erfasst werden können.

Vorzugsweise ist der mindestens eine Ultraschallwandler ein piezoelektrischer Wandler, der Ultraschallsignale abgeben und empfangen kann und mit der Steuer- und Auswerteeinheit verbunden ist. Ein derartiger piezoelektrischer Wandler hat den Vorteil, dass er gleichermaßen zum Abgeben von Ultraschallsignalen dienen kann, also als Lautsprecher, sowie auch als Empfänger von Ultraschallsignalen, also als Mikrofon. Darüber hinaus haben piezoelektrische Wandler den Vorteil, dass sie effizient sind und in einem gewünschten Schallfrequenzbereich betrieben werden können.

Die Steuer- und Auswerteeinheit weist vorzugsweise einen Speicher auf oder ist mit einem solchen verbunden. In dieser Variante kann in dem Speicher ein Referenzsignal gespeichert sein, und die Steuer- und Auswerteeinheit kann dazu ausgebildet sein, ein jeweiliges empfangenes Ultraschallsignal mit dem Referenzsignal zu vergleichen, um so eine Lageänderung des Implantats und/oder einer weiteren Komponente des Therapie- und/oder Monitoringsystems zu erkennen. Das gespeicherte Referenzsignal kann vorgegeben sein oder beispielsweise zu einem Zeitpunkt aufgenommen sein, an dem sich das Implantat oder eine weitere Komponente des Therapie- und/oder Monitoringsystems an einem gewünschten Ort befinden. Wenn ein später empfangenes Ultraschallsignal vom Referenzsignal stark abweicht, ist dies ein Hinweis darauf, dass sich die Lage des Implantats und/oder der weiteren Komponente des Therapie- und/oder Monitoringsystems geändert hat. Insbesondere kann das Implantat einen oder mehrere Ultraschallwandler aufweisen, und die Steuer- und Auswerteeinheit kann zunächst eine für den Implantationsort übliche Ultraschallreferenz ("Ultraschall-Fingerabdruck") aufzeichnen und abspeichern und diese dann mit zyklischen Messungen vergleichen. Weicht die aktuelle Messung vom Referenzmuster über ein gewisses Maß hinaus ab, zeigt das Implantat eine mögliche Lageveränderung an. So kann auch ein Verdrehen eines Implantates erkannt werden.

Es kann vorteilhaft sein, wenn das Therapie- und/oder Monitoringsystem mehrere Ultraschallwandler zum Aussenden und Empfangen von Ultraschallsignalen aufweist, die unbeweglich mit dem Implantat oder der weiteren Komponente des Therapie- und/oder Monitoringsystems verbunden sind. In diesem Fall ist die Steuer- und Auswerteeinheit vorzugsweise dazu ausgebildet, zu einem ersten Zeitpunkt von dem Ultraschallwandler empfangene Ultraschallsignale als Referenzsignale zu speichern und zu einem oder mehreren späteren Zeitpunkten von dem Ultraschallwandler empfangene Ultraschallsignale mit den Referenzsignalen zu vergleichen und auf Basis dieses Vergleichs zu bestimmen, ob Unterschiede zwischen jeweils aktuell empfangenen Ultraschallsignalen und den Referenzsignalen eine Lageänderung des Implantats und/oder der weiteren Komponente des Therapie- und/oder Monitoringsystems indizieren.

Das Therapie- und/oder Monitoringsystem kann auch mindestens zwei Ultraschallwandler aufweisen, die derart angebracht sind, dass die Position eines Ultraschallechos oder Ultraschallsignals flächig oder räumlich zugeordnet werden kann (2D- 3D-Ultraschallhören) .

Das Therapie- und/oder Monitoringsystem ist ein Herztherapiesystem, das neben dem Implantat als weitere Komponente eine Elektrodenleitung aufweist, die mit dem Implantat verbunden ist. In diesem Falle ist die Steuer- und Auswerteeinheit dazu ausgebildet, eine Lageänderung der Elektrodenleitung, und insbesondere eines oder mehrerer Elektrodenpole der Elektrodenleitung, zu erfassen. Dementsprechend ist der wenigstens eine Ultraschallwandler an oder in einer Elektrodenleitung, und zwar nahe einem Elektrodenpol der Elektrodenleitung, angeordnet.

Im Falle eines Therapie- und/oder Monitoringsystems in Form eines Herztherapiesystems mit einer Elektrodenleitung ist es vorteilhaft, wenn wenigstens zwei Ultraschallwandler vorgesehen sind, von denen wenigstens einer an oder in dem Implantat, also beispielsweise dem Herzschrittmacher, angeordnet ist, während der wenigstens zweite Ultraschallwandler an oder in der Elektrodenleitung angeordnet ist und beide Ultraschallwandler derart mit der Steuer- und Auswerteeinheit verbunden sind, dass einer der wenigstens zwei Ultraschallwandler durch die Steuer- und Auswerteeinheit ausgelöste Ultraschallsignale aussendet, während der jeweils wenigstens eine andere Ultraschallwandler diese Ultraschallsignale empfängt. Hierbei ist die Steuer- und Auswerteeinheit vorzugsweise dazu ausgebildet, eine Signallaufzeit zwischen dem Aussenden eines Ultraschallsignals durch den einen der Ultraschallwandler und Empfangen des Ultraschalls durch den jeweils anderen der Ultraschallwandler zu erfassen und auszuwerten. Auf diese Weise kann die Steuer- und Auswerteeinheit über die Signallaufzeit, gegebenenfalls unter Berücksichtigung der Phasenlage des Signals, leicht Abstandsänderungen zwischen Implantat und Elektrodenleitung feststellen, die einen Hinweis auf eine Dislokation der Elektrodenleitung liefern können. Das implantierbare System misst dann die Ultraschallaufzeit von der Elektrode zum Implantatgehäuse oder umgekehrt und wertet optional dessen zeitlichen Verlauf aus, so dass z.B. auch die Bewegung einer im Herzen positionierten Elektrode bewertet werden kann.

Alternativ oder zusätzlich ist es auch möglich, dass wenigstens einer in oder an der Elektrodenleitung angeordneter Ultraschallwandler mit der Steuer- und Auswerteeinheit derart verbunden ist, dass er auf ein Signal der Steuer- und Auswerteeinheit hin ein Ultraschallsignal aussendet und reflektierte Schallsignalanteile des ausgesendeten Ultraschallsignals empfängt, wobei die Steuer- und Auswerteeinheit dazu ausgebildet ist, die reflektierten Ultraschallsignale auszuwerten. Auf diese Weise ist es beispielsweise mit Hilfe nur eines einzigen Ultraschallwandlers in der Elektrodenleitung möglich, einen Abstand zwischen der Elektrodenleitung und einem Implantat oder anderen Strukturen auf Basis reflektierter Ultraschallsignalanteile zu bestimmen. Beispielsweise befindet sich der Ultraschallwandler an der Elektrode und misst die typische Reflexion der Ultraschallwellen am Implantatgehäuse zur Lagebestimmung. Es sei darauf hingewiesen, dass auch in diesem Fall zwei Ultraschallwandler vorgesehen sein können, die beispielsweise beide in der Elektrodenleitung angeordnet sind und von denen einer Ultraschallsignale aussendet, während der andere die reflektierten Signalanteile empfängt.

Grundsätzlich kann die Steuer- und Auswerteeinheit dazu ausgebildet sein, empfangene Ultraschallsignale hinsichtlich Laufzeit, Frequenzverschiebung, Amplitude und/oder Phasenlage auszuwerten.

Insbesondere ist es vorteilhaft, wenn die Steuer- und Auswerteeinheit ausgebildet ist, Frequenzverschiebungen zwischen der Frequenz empfangener Ultraschallsignale und der Frequenz ausgesandter Ultraschallsignale zu erfassen, um das Vorhandensein oder Nichtvorhandensein eines Dopplereffektes zu bestimmen, also beispielsweise zu bestimmen, ob sich beispielsweise eine Elektrodenleitung in einem strömenden Medium, wie Blut, befindet. Die Steuer- und Auswerteeinheit ist vorzugsweise ausgebildet, den Dopplereffekt auszuwerten, um so zu bestimmen, ob das Implantat noch immer an einem sich bewegenden Zielgewebe fixiert ist oder sich in bzw. an einer Blutstrombahn befindet. Alternativ oder zusätzlich kann die Steuer- und Auswerteeinheit ausgebildet sein, den Dopplereffekt so auszuwerten, dass die Steuer- und Auswerteeinheit feststellt, ob sich das Implantat nicht in einer Blutstrombahn befindet.

In dem Fall, in dem das Therapie- und/oder Monitoringsystem einen Herzstimulator und/oder einen Herzmonitor als Implantat umfasst, ist die Steuer- und Auswerteeinheit ausgebildet, empfangene Ultraschallsignale unter Berücksichtigung von von dem Implantat aufgenommenen und einen jeweiligen Herzzyklus repräsentierenden physiologischen Signalen auszuwerten. Derartige physiologische Signale können beispielsweise intrakardiale Elektrokardiogramme sein, die den Herzzyklus repräsentieren. In diesem Fall ist es möglich, Bewegungen innerhalb einer Herzkammer, die auch zu zyklischen Lageänderungen der jeweiligen Elektrodenleitung führen, mit den elektrophysiologischen Signalen des Herzens zu korrelieren und so festzustellen, dass die per Ultraschall gemessenen Lageänderungen der zu erwartenden Lageänderungen infolge der Bewegung des Herzens entsprechen, so dass dies ein Indiz auf eine nicht dislokalisierte Elektrodenleitung ist.

In diesem Zusammenhang ist es insbesondere vorteilhaft, wenn die Steuer- und Auswerteeinheit ausgebildet ist, aus empfangenen Ultraschallsignalen ein Bewegungsprofil des Implantats zu generieren und ein solches Bewegungsprofil in Relation zu einen Herzzyklus repräsentierenden elektrophysiologischen Signalen auszuwerten. Auf diese weise wird die Ultraschallauswertung eines am oder in der Blutstrombahn befindlichen Implantates um die Auswertung des Herzzyklus (EKG-Einheit) ergänzt, um das Bewegungsprofil der Blutströmung oder des Implantates hinsichtlich möglicher Dislokationen auswerten zu können (z.B. die Dislokation eines rechtsventrikulären Leadless-Pacemakers in die Pulmonalarterie oder das Atrium).

Die Erfindung soll nun anhand von Ausführungsbeispielen mit Bezug auf die Figuren näher erläutert werden. Die Figuren zeigen in
- Figur 1:: ein schematisches Blockschaltbild eines erfindungsgemäßen Implantates;
- Figur 2:: ein erstes Applikationsbeispiel eines Dislokationssensors für eine Ultraschall-Dopplermessung im Blutgefäß;
- Figur 3:: ein weiteres Implementierungsbeispiel für eine Laufzeitmessung;
- Figur 4:: eine weitere Ausführungsform der Dislokationserkennung für ein Ultraschall-Stereo-Hören; und
- Figur 5:: einen einfachen Dislokationssensor mit Ultraschallreferenz

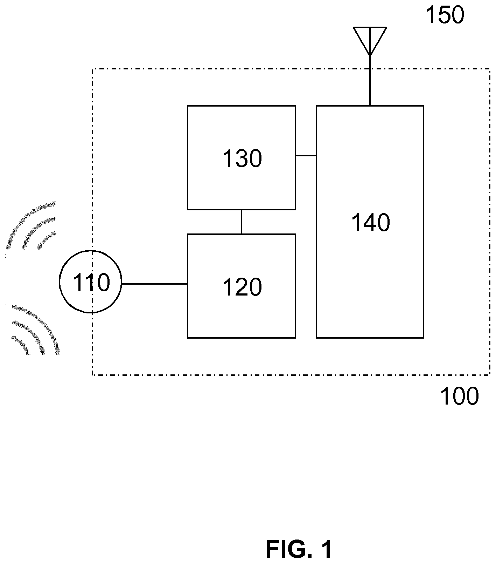

In Figur 1 ist ein aktives implantierbares medizinisches Therapie- und/oder Monitoringsystem in Form eines Implantats 100 abgebildet. Die Figur zeigt das Blockschaltbild des Implantates 100. Dieses beinhaltet einen oder mehrere Ultraschallwandler 110, angebracht am Gehäuse des Implantates 100 oder aber als Bestandteil einer an das Implantat 100 angeschlossenen Elektrodenleitung. Der Ultraschallwandler 110 ist mit einer Ultraschall-Sende- und Empfangseinheit 120 verbunden, welche wiederum an eine Steuer- und Auswerteeinheit 130 angeschlossen ist. Diese Steuer- und Auswerteeinheit 130 startet zyklisch oder auf bestimmte Ereignisse getriggert einen Ultraschall-Scan und wertet die empfangenen Ultraschallsignale dahingehend aus, die aktuelle Position des Implantates oder der Elektrodenleitung zu bewerten. Die Bewertung kann dabei anhand von Ultraschallaufzeiten, Frequenzverschiebungen (Dopplereffekt), Amplitudenwerten und Phasenlagen erfolgen. Eine zusätzliche Telemetrieeinheit 140 mit Antenne 150 ist wiederum mit der Steuer- und Auswerteeinheit 130 verbunden, so dass eine erkannte Dislokation entsprechend signalisiert werden kann.

Einer Therapie oder einem Monitoring dienende Einheiten des Implantats sind in Figur 1 nicht dargestellt und für sich genommen bekannt.

Die einzusetzenden Ultraschallfrequenzen sollten im Bereich von ∼1MHz liegen, so dass eine Eindringtiefe von ca. 50cm realisiert wird. Allerdings sind hier applikationsspezifische Unterschiede zu berücksichtigen.

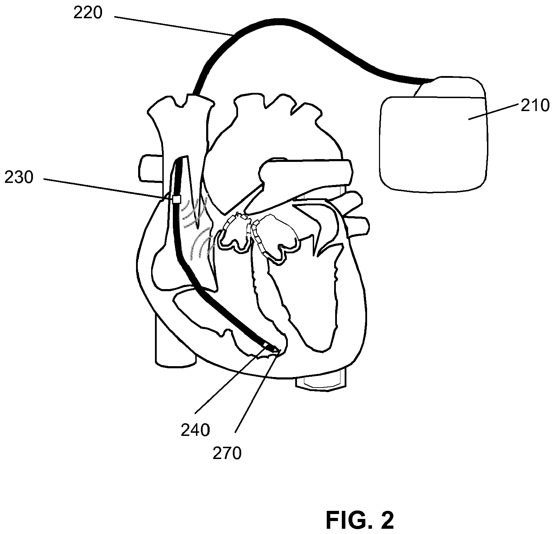

In Figur 2 ist ein erstes Applikationsbeispiel eines Dislokationssensors dargestellt. Hier ist ein implantierbarer Herzschrittmacher 210 mit einer implantierbaren Elektrodenleitung 220 verbunden, welche zusätzlich einen Ultraschallwandler 230 beinhaltet. Außerdem befindet sich zum bestimmungsgemäßen Gebrauch im distalen Bereich der Elektrode ein Stimulationselektrodenpol bestehend aus einer Ringelektrode 240 und einer Tip-Elektrode 270.

In diesem Beispiel ist der Ultraschallwandler 230 nebst zugehöriger Ultraschall-Sende- oder Empfangseinheit und Steuer- und Auswerteeinheit derart konfiguriert, dass dieser mittels einer Herzfrequenz-synchronen Messung und Frequenzauswertung (Dopplereffekt) den Blutstrom in der Vena cava nachweist und damit die Position der Elektrode bestätigt.

Eine solche Messung kann insbesondere auch für die Dislokationserkennung von Pulmonalarterien-Drucksensoren angewendet werden.

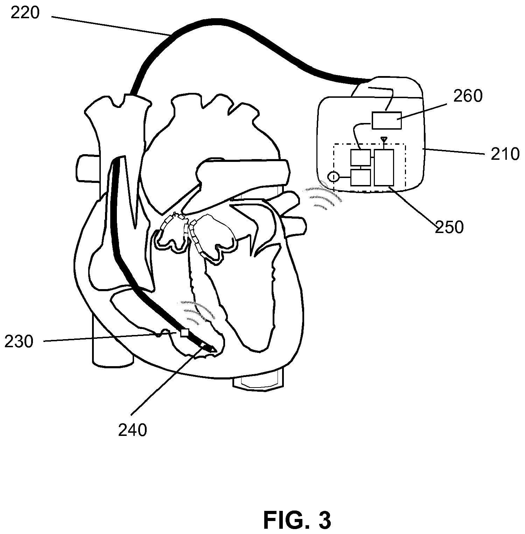

Figur 3 zeigt ein weiteres Implementierungsbeispiel. Das Implantat 210 ist mit einer Elektrodenleitung 220 verbunden, die einen Mini-Piezo-Ultraschallsender 230 und einen Stimulationselektrodenpol 240 umfasst. Der Ultraschallsender 230 ist in der Nähe des Stimulationselektrodenpols 240 angebracht. Zusätzlich ist am Implantatgehäuse 210 ein Ultraschallempfänger 250 montiert.

Zur Bewertung der Position des Stimulationselektrodenpols 240 wird nun die Ultraschalllaufzeit zwischen Sender 240 und Empfänger 250 ausgewertet. Verändert sich diese über ein zulässiges Maß hinaus, wird eine Dislokation der Elektrodenspitze angezeigt.

Zur Verbesserung der Spezifität und Sensitivität kann die Laufzeitmessung kontinuierlich über einen Herzzyklus ausgeführt werden und aus der so gewonnenen Bewegungskurve eine Differenzierung einer sogenannten Mikrodislokation mit zusätzlichen sekundären Elektrodenbewegungen (unphysiologisches "Wackeln" der Elektrodenspitze) vorgenommen werden.

Hierzu ist das Implantat 210 mit einer Erfassungseinheit 260 zum Aufnehmen eines intrakardialen Elektrokardiogramms ausgestattet, die ein über den Elektrodenpol 240 oder 270 aufgenommenes elektrophysiologisches Signal auswertet.
Der Empfänger 250 entspricht beispielsweise dem Ultraschallwandler 110 und der Ultraschall-Sende- und Empfangseinheit 120 aus Figur 1. Die Steuer- und Auswerteeinheit 130 ist mit der Erfassungseinheit 260 verbunden.

Alternativ können der sendende Ultraschallwandler und der empfangende Ultraschallwandler getauscht werden, d.h. Sender ist der Ultraschallwandler 250 und Empfänger der Ultraschallwandler 230.

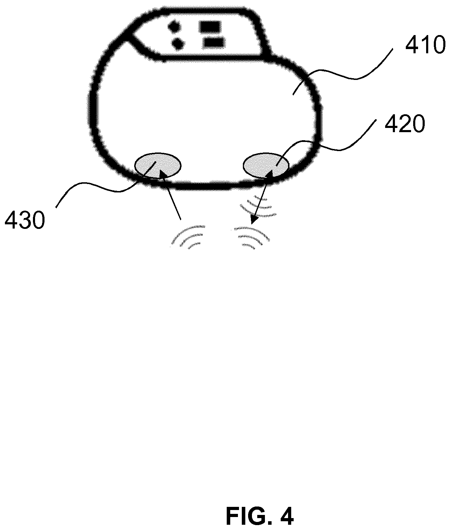

In Figur 4 ist eine weitere Ausführungsform der Dislokationserkennung dargestellt. Hier sind am Gehäuse eines Implantates 410 mindestens zwei Piezo-Ultraschallwandler 420 und 430 nebst zugehöriger Ultraschall-Sende- oder Empfangseinheit angebracht, wobei der erste Ultraschallwandler 420 zum Aussenden eines Ultraschallimpulses genutzt wird, dessen Reflektionen dann von dem ersten Ultraschallwandler 420 und dem zweiten Ultraschallwandler 430 empfangen werden. Aus der Differenzbetrachtung beider Empfänger kann dann eine 2-dimensionale Klassifikation der Ultraschallreflektion erfolgen. So kann zum Beispiel das typische Echo einer Reflektionsfläche an einer Elektrode vermessen werden.

Das Prinzip kann optional auf weitere Empfänger ausgedehnt werden (z.B. 3-dimensional). Ebenso können die Ultraschallwandler in der Elektrodenleitung implementiert werden und das Gehäuse 410 dient als Reflektor für den Ultraschall. Vorteil dieser Anordnung ist die Möglichkeit, die Ultraschallwandler hinreichend weit voneinander zu positionieren und ein sehr großes, charakteristisches Echo vom Implantatgehäuse 410 aufzuzeichnen. Ein weiterer Vorteil ist die Möglichkeit, die Position mehrere Elektrodenabschnitte gleichzeitig zu überprüfen.

Optional kann (hier nicht dargestellt) ein separater Echoreflektor als Positionsreferenz implantiert werden.

Das Prinzip kann auch mit dem in Figur 3 erläuterten kombiniert werden. Dann enthält z.B. die Elektrodenleitung mindestens einen Mini-Piezo-Ultraschallsender (230 - siehe Figur 3) und am Implantat sind mindestens zwei Piezo-Ultraschallempfänger (420, 430 - siehe Figur 4) angebracht, mit deren Hilfe dann durch Auswertung von Laufzeit- und Amplitudenunterschieden sowie unter Nutzung des Dopplereffektes durch die sich bewegende Elektrode eine Verbesserung der Spezifität und Sensitivität der Dislokationserkennung ermöglicht werden kann. Ultraschallsender 230 und Ultraschallempfänger 420 und 430 weisen jeweils einen Ultraschallwandler wie den Ultraschallwandler 110 aus Figur 1 auf sowie eine Ultraschall-Sende- oder Empfangseinheit wie die Ultraschall-Sende- und Empfangseinheit 120 aus Figur 1.

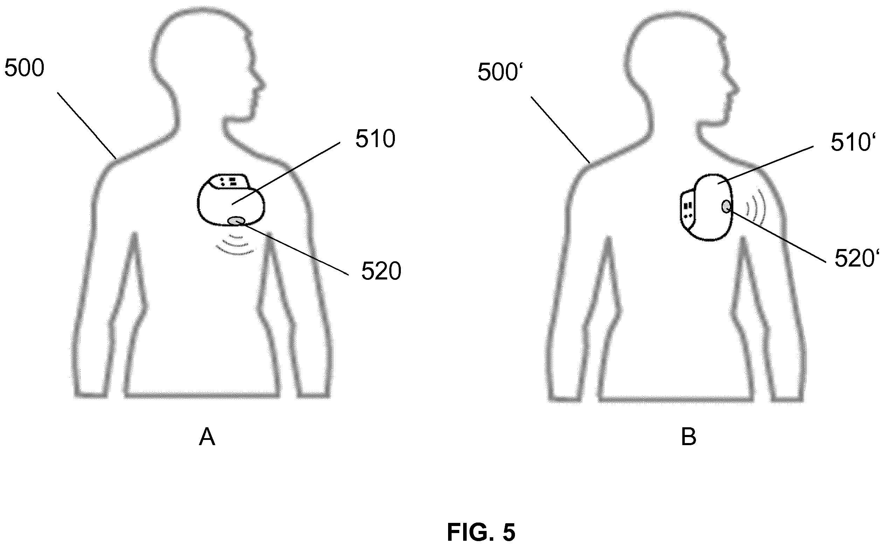

Figur 5 zeigt einen Dislokationssensor, der mit nur einem Ultraschallwandler 520 auf dem Implantat 510 realisiert werden kann. Dieser Ultraschallwandler 520 ist Teil eines Sensors mit einer Ultraschall-Sende- und Empfangseinheit 120 und einer Steuer- und Auswerteeinheit 130, die bei bekannter Implantatsposition im Patienten (500, A) eine Ultraschallreflektions-Referenz aufzeichnet und diese abspeichert. Für die spätere Implantatüberwachung werden zyklisch weitere Ultraschallmessungen durchgeführt und mit der aufgezeichneten Referenz verglichen. Überschreiten die Abweichungen von der Referenz einen Grenzwert, so wird eine Dislokation (B) angenommen und signalisiert. Ein solcher Aufbau kann besonders dann genutzt werden, wenn ein Verdrehen eines Implantates (z.B. ein Herzmonitor) erkannt werden soll.

Weitere aktive Implantate mit dem beschriebenen Ultraschalldislokationssensor können sein, Insulinpumpen, Herzunterstützungspumpen (VAD), implantierbare Monitore, Pill-Cam Implantate, Neurostimulatoren, Stimulatoren zur kardialen Resynchronisation, implantierbare Steuerungen für orthopädische Implantate und Retinaimplantate.

Ebenso ist die Ultraschallüberwachung der Implantatpositionierung geeignet, um eine aktive Überwachung von bislang nur passiven Implantaten, wie z.B. orthopädische Prothesen zu realisieren.

Die derzeit in kommerziell verfügbaren medizinischen Ultraschalldiagnosesystemen (3D- und 4D-Echokardiographie mit Matrix-Technologie) eingesetzten Ultraschallwandler, können auf eine Größe von etwa 350µm miniaturisiert werden und sind damit ohne Einschränkungen auch für alle genannten Implantatanwendungen geeignet.

### Bezugszeichenliste

- 100: Implantat
- 110: Ultraschallwandler
- 120: Ultraschall-Sende- und Empfangseinheit
- 130: Steuer- und Auswerteeinheit
- 140: Telemetrieeinheit
- 150: Antenne
- 210: Herzschrittmacher
- 220: Elektrodenleitung
- 230: Ultraschallwandler / Ultraschalltransducer
- 240: Stimulationselektrodenpol
- 250: Ultraschallempfänger
- 260: Elektrogrammerfassungseinheit
- 270: Tip-Elektrodenpol
- 410: Implantat
- 420: Ultraschallwandler
- 430: Ultraschallwandler
- 500: Patient
- 510: Implantat
- 520: Ultraschallwandler

## Patentansprüche

1. Aktives implantierbares medizinisches Therapie- und/oder Monitoringsystem, das eine Steuer- und Auswerteeinheit (130) und mindestens ein Implantat (100) umfasst, wobei das Implantat (100) wenigstens einen Ultraschallwandler (230) zum Abgeben und Aufnehmen von Ultraschallsignalen umfasst, der direkt oder indirekt mit der Steuer- und Auswerteeinheit (130) verbunden ist, wobei die Steuer-und Auswerteeinheit (130) ausgebildet ist, ein Abgeben von Ultraschallsignalen durch den Ultraschallwandler (230) zyklisch oder getriggert auszulösen und empfangene Ultraschallsignale so auszuwerten, dass die Steuer- und Auswerteeinheit (130) eine jeweils aktuelle Lage oder Lageänderung des Implantates (100) und/oder eine weitere Komponente (220) des aktiven implantierbaren medizinischen Therapie- und/oder Monitoringsystem erkennt,
wobei die Steuer- und Auswerteeinheit (130) einen Speicher aufweist oder mit einem Speicher verbunden ist, in dem ein Referenzsignal gespeichert ist, wobei die Steuer- und Auswerteeinheit (130) ausgebildet ist, ein jeweiliges empfangenes Ultraschallsignal mit dem Referenzsignal zu vergleichen, um so eine Lageänderung des Implantats (100) und/oder einer weiteren Komponente des Therapie- und/oder Monitoringsystems zu erkennen,
**dadurch gekennzeichnet, dass**
die Steuer- und Auswerteeinheit (130) dazu ausgelegt ist, eine für einen Implantationsort übliche Ultraschallreferenz aufzuzeichnen, abzuspeichern und die Ultraschallreferenz mit den empfangenen Ultraschallsignalen zu vergleichen.

2. Therapie- und/oder Monitoringsystem gemäß Anspruch 1, **dadurch gekennzeichnet, dass** der wenigstens eine Ultraschallwandler (230) ein piezoelektrischer Wandler ist, der Ultraschallsignale abgeben und empfangen kann und mit der Steuer- und Auswerteeinheit (130) verbunden ist.

3. Therapie- und/oder Monitoringsystem gemäß wenigstens einem der Ansprüche 1 oder 2, **dadurch gekennzeichnet, dass** das Therapie- und/oder Monitoringsystem ein Herztherapiesystem ist und als sonstige Komponente neben dem Implantat (100) eine Elektrodenleitung (220) aufweist.

4. Therapie- und/oder Monitoringsystem gemäß Anspruch 3, **dadurch gekennzeichnet, dass** der wenigstens eine Ultraschallwandler (230) an oder in der Elektrodenleitung (220), vorzugsweise nahe einem Elektrodenpol der Elektrodenleitung (220), angeordnet ist.

5. Therapie- und/oder Monitoringsystem gemäß Anspruch 3 oder 4, **dadurch gekennzeichnet, dass** wenigstens ein zweiter Ultraschallwandler (230) in oder an dem Implantat (100) angeordnet ist und dass der wenigstens eine Ultraschallwandler (230) an oder in der Elektrodenleitung (220) derart mit der Steuer- und Auswerteeinheit (130) verbunden ist, dass er, durch diese ausgelöst, Ultraschallsignale aussendet, während der wenigstens eine zweite Ultraschallwandler (110) in dem Implantat (100) dazu ausgebildet ist, Ultraschallsignale zu empfangen oder umgekehrt, wobei die Steuer- und Auswerteeinheit (130) ausgebildet ist, eine Signallaufzeit zwischen dem Aussenden eines Ultraschallsignals durch einen der Ultraschallwandler (110, 230) und Empfangen des Ultraschallsignals durch einen jeweils anderen der Ultraschallwandler (230, 110) zu erfassen und auszuwerten.

6. Therapie- und/oder Monitoringsystem gemäß Anspruch 4, **dadurch gekennzeichnet, dass** der wenigstens eine in oder an der Elektrodenleitung (220) angeordnete Ultraschallwandler (230) mit der Steuer- und Auswerteeinheit (130) derart verbunden ist, dass er auf ein Signal der Steuer- und Auswerteeinheit (130) hin ein Ultraschallsignal aussendet und reflektierte Ultraschallsignalanteile des ausgesendeten Ultraschallsignals empfängt, wobei die Steuer- und Auswerteeinheit (130) dazu ausgebildet ist, die reflektierten Ultraschallsignalanteile auszuwerten.

7. Therapie- und/oder Monitoringsystem gemäß wenigstens einem der Ansprüche 1 bis 6, **dadurch gekennzeichnet, dass** das Therapie- und/oder Monitoringsystem mehrere Ultraschallwandler (230) zum Aussenden und Empfangen von Ultraschallsignalen aufweist, die unbeweglich mit dem Implantat (100) verbunden sind, wobei die Steuer- und Auswerteeinheit (130) ausgebildet ist, zu einem ersten Zeitpunkt von den Ultraschallwandlern (230) empfangene Ultraschallsignale als Referenzsignale zu speichern und zu einem oder mehreren späteren Zeitpunkten von den Ultraschallwandlern (230) empfangene Ultraschallsignale mit den Referenzsignalen zu vergleichen und auf Basis des Vergleichs zu bestimmen, ob Unterschiede zwischen jeweils aktuell empfangenen Ultraschallsignalen und den Referenzsignalen eine Lageänderung des Implantats (100) indizieren.

8. Therapie- und/oder Monitoringsystem gemäß wenigstens einem der Ansprüche 1 bis 7, **dadurch gekennzeichnet, dass** die Steuer- und Auswerteeinheit (130) ausgebildet ist, empfangene Ultraschallsignale hinsichtlich Laufzeit, Frequenzverschiebung, Amplitude und/oder Phasenlage auszuwerten.

9. Therapie- und/oder Monitoringsystem gemäß wenigstens einem der Ansprüche 1 bis 8, **dadurch gekennzeichnet, dass** die Steuer- und Auswerteeinheit (130) ausgebildet ist, Frequenzverschiebungen empfangener Ultraschallsignale gegenüber der Frequenz ausgesandter Ultraschallsignale zu erfassen, um das Vorhandensein oder Nichtvorhandensein eines Dopplereffektes zu bestimmen.

10. Therapie- und/oder Monitoringsystem gemäß wenigstens einem der Ansprüche 1 bis 9, **dadurch gekennzeichnet, dass** das Implantat (100) ein Herzstimulator und/oder ein Herzmonitor ist und dass die Steuer- und Auswerteeinheit (130) ausgebildet ist, empfangene Ultraschallsignale unter Berücksichtigung von von dem Implantat (100) aufgenommenen und einen jeweiligen Herzzyklus repräsentierenden physiologischen Signalen auszuwerten.

11. Therapie- und/oder Monitoringsystem gemäß Anspruch 10, **dadurch gekennzeichnet, dass** die Steuer- und Auswerteeinheit (130) ausgebildet ist, aus empfangenen Ultraschallsignalen ein Bewegungsprofil des Implantats zu generieren und ein solches Bewegungsprofil in Relation zu einen Herzzyklus repräsentierenden elektrophysiologischen Signalen auszuwerten.

12. Therapie- und/oder Monitoringsystem gemäß wenigstens einem der Ansprüche 1 bis 11, **dadurch gekennzeichnet, dass** die von dem Implantat aufgenommenen und einen jeweiligen Herzzyklus repräsentierenden physiologischen Signale intrakardiale Elektrokardiogramme sind.

## Claims

1. An active implantable medical therapy and/or monitoring system comprising at least one control and evaluation unit (130) and at least one implant (100), wherein the implant (100) comprises at least one ultrasonic transducer (230) for emitting and recording ultrasonic signals, which ultrasonic transducer is directly or indirectly connected to the control and evaluation unit (130), wherein the control and evaluation unit (130) is designed to prompt an emission of ultrasonic signals by the ultrasonic transducer (230) cyclically or in a triggered manner and to evaluate received ultrasonic signals such that the control and evaluation unit (130) identifies an actual position or change in position of the implant (100) and/or a further component (220) of the active implantable medical therapy and/or monitoring system,
wherein the control and evaluation unit (130) has a memory or is connected to a memory, in which a reference signal is stored, wherein the control and evaluation unit (130) is designed to compare a received ultrasonic signal with the reference signal in order to thus identify a change in position of the implant (100) and/or a further component of the therapy and/or monitoring system,
**characterised in that**
the control and evaluation unit (130) is designed to record an ultrasonic reference that is usual for an implantation site and to compare the ultrasonic reference with the received ultrasonic signals.

2. The therapy and/or monitoring system according to claim 1, **characterised in that** the at least one ultrasonic transducer (230) is a piezoelectric transducer that may emit and receive ultrasonic signals and that is connected to the control and evaluation unit (130).

3. The therapy and/or monitoring system according to at least one of claims 1 or 2, **characterised in that** the therapy and/or monitoring system is a heart therapy system and comprises an electrode line (220) as another component in addition to the implant (100).

4. The therapy and/or monitoring system according to claim 3, **characterised in that** the at least one ultrasonic transducer (230) is arranged on or in the electrode line (220), preferably close to an electrode pole of the electrode line (220).

5. The therapy and/or monitoring system according to claim 3 or 4, **characterised in that** at least one second ultrasonic transducer (230) is arranged in or on the implant (100), and **in that** the at least one ultrasonic transducer (230) is connected on or in the electrode line (220) to the control and evaluation unit (130) in such a way that the at least one ultrasonic transducer, triggered by said control and evaluation unit, emits ultrasonic signals, whilst the at least one second ultrasonic transducer (110) in the implant (100) is designed to receive ultrasonic signals or vice versa, wherein the control and evaluation unit (130) is designed to detect and evaluate a signal propagation time between an emission of an ultrasonic signal by one of the ultrasonic transducers (110, 230) and receipt of the ultrasonic signal by another of the ultrasonic transducers (230, 110).

6. The therapy and/or monitoring system according to claim 4, **characterised in that** the at least one ultrasonic transducer (230) arranged in or on the electrode line (220) is connected to the control and evaluation unit (130) in such a way that the at least one ultrasonic transducer emits an ultrasonic signal in response to a signal of the control and evaluation unit (130) and receives reflected ultrasonic signal components of the emitted ultrasonic signal, wherein the control and evaluation unit (130) is designed to evaluate the reflected ultrasonic signal components.

7. The therapy and/or monitoring system according to at least one of claims 1 to 6, **characterised in that** the therapy and/or monitoring system comprises a plurality of ultrasonic transducers (230) for emitting and receiving ultrasonic signals, which plurality of ultrasonic transducers are connected immovably to the implant (100), wherein the control and evaluation unit (130) is designed to store, as reference signals, ultrasonic signals received by the ultrasonic transducers (230) at a first moment in time and to compare ultrasonic signals received by the ultrasonic transducers (230) at one or more later moments in time with the reference signals, and to determine, based on the comparison, whether differences between currently received ultrasonic signals and the reference signals indicate a change in position of the implant (100).

8. The therapy and/or monitoring system according to at least one of claims 1 to 7, **characterised in that** the control and evaluation unit (130) is designed to evaluate received ultrasonic signals in terms of propagation time, frequency shift, amplitude and/or phase position.

9. The therapy and/or monitoring system according to at least one of claims 1 to 8, **characterised in that** the control and evaluation unit (130) is designed to detect frequency shifts of received ultrasonic signals compared to the frequency of emitted ultrasonic signals to determine the presence or absence of a Doppler effect.

10. The therapy and/or monitoring system according to at least one of claims 1 to 9, **characterised in that** the implant (100) is a heart stimulator and/or a heart monitor, and **in that** the control and evaluation unit (130) is designed to evaluate received ultrasonic signals under consideration of physiological signals that are received by the implant (100) and that represent a heart cycle.

11. The therapy and/or monitoring system according to claim 10, **characterised in that** the control and evaluation unit (130) is designed to generate a movement profile of the implant from received ultrasonic signals and to evaluate such a movement profile in relation to electrophysiological signals representing a heart cycle.

12. The therapy and/or monitoring system according to at least one of claims 1 to 11, **characterised in that** the physiological signals received by the implant and representing a heart cycle are intracardiac electrocardiograms.

## Revendications

1. Système implantable de thérapie et/ou de surveillance médicale active qui comprend une unité de commande et d'exploitation (130) et au moins un implant (100), dans lequel l'implant (100) comprend au moins un transducteur à ultrasons (230), permettant l'émission et la réception de signaux ultrasonores, qui est relié directement ou indirectement avec l'unité de commande et d'exploitation (130), dans lequel l'unité de commande et d'exploitation (130) est conçue pour provoquer de manière cyclique ou déclenchée une émission de signaux ultrasonores par le transducteur à ultrasons (230) et pour évaluer des signaux ultrasonores reçus de telle manière que l'unité de commande et d'exploitation (130) reconnaît respectivement une position actuelle ou une modification de la position de l'implant (100) et/ou d'un autre composant (220) du système implantable de thérapie et/ou de surveillance médicale active,
dans lequel l'unité de commande et d'exploitation (130) présente une mémoire ou est reliée avec une mémoire dans laquelle est stocké un signal de référence, dans lequel l'unité de commande et d'exploitation (130) est conçue pour comparer un signal ultrasonore reçu respectif avec le signal de référence, afin de reconnaître ainsi la modification de position de l'implant (100) et/ou d'un autre composant du système de thérapie et/ou de surveillance,
**caractérisé en ce que**
l'unité de commande et d'exploitation (130) est conçue pour enregistrer, stocker une référence ultrasonore usuelle pour un lieu d'implantation et comparer la référence ultrasonore avec les signaux ultrasonores reçus.

2. Système de thérapie et/ou de surveillance selon la revendication 1, **caractérisé en ce que** l'au moins un transducteur à ultrasons (230) est un transducteur piézoélectrique qui peut émettre et recevoir des signaux ultrasonores et est relié avec l'unité de commande et d'exploitation (130).

3. Système de thérapie et/ou de surveillance selon au moins une des revendications 1 ou 2, **caractérisé en ce que** le système de thérapie et/ou de surveillance est un système de thérapie cardiaque et présente en tant qu'autre composant à côté de l'implant (100) une ligne d'électrode (220).

4. Système de thérapie et/ou de surveillance selon la revendication 3, **caractérisé en ce que** l'au moins un transducteur à ultrasons (230) est disposé sur ou dans la ligne d'électrode (220), de préférence près d'un pôle d'électrode de la ligne d'électrode (220).

5. Système de thérapie et/ou de surveillance selon la revendication 3 ou la revendication 4, **caractérisé en ce qu'**au moins un deuxième transducteur à ultrasons (230) est disposé dans ou sur l'implant (100), et que l'au moins un transducteur à ultrasons (230) sur ou dans la ligne d'électrode (220) est relié de telle manière avec l'unité de commande et d'exploitation (130) qu'il émet des signaux ultrasonores provoqués par celle-ci, tandis que l'au moins un deuxième transducteur à ultrasons (110) dans l'implant (100) est conçu pour recevoir des signaux ultrasonores, ou inversement, où l'unité de commande et d'exploitation (130) est conçue pour détecter et exploiter un temps de propagation de signal entre l'émission d'un signal ultrasonore par le transducteur à ultrasons (110, 230) et la réception du signal ultrasonore par respectivement l'autre des transducteurs à ultrasons (230, 110.

6. Système de thérapie et/ou de surveillance selon la revendication 4, **caractérisé en ce que** l'au moins un transducteur à ultrasons (230) disposé dans ou sur la ligne d'électrode (220) est relié avec l'unité de commande et d'exploitation (130) de telle manière qu'il émet un signal ultrasonore suite à un signal de l'unité de commande et d'exploitation (130) et reçoit des parties de signal ultrasonore réfléchies du signal ultrasonore émis, où l'unité de commande et d'exploitation (130) est conçue pour évaluer les parties de signaux ultrasonores réfléchies.

7. Système de thérapie et/ou de surveillance selon au moins une des revendications 1 à 6, **caractérisé en ce que** le système de thérapie et/ou de surveillance présente plusieurs transducteurs à ultrasons (230) pour l'émission et la réception de signaux ultrasonores qui sont reliés non mobiles avec l'implant (100), où l'unité de commande et d'exploitation (130) est conçue pour enregistrer, dans un premier temps, des signaux ultrasonores reçus par les transducteurs à ultrasons (230) sous forme de signaux de référence et, dans un ou plusieurs temps ultérieurs, pour comparer des signaux ultrasonores reçus par les transducteurs à ultrasons (230) avec les signaux de référence et, sur la base de la comparaison, déterminer si des différences entre respectivement des signaux ultrasonores reçus actuellement et les signaux de référence indiquent une modification de position de l'implant (100).

8. Système de thérapie et/ou de surveillance selon au moins une des revendications 1 à 7, **caractérisé en ce que** l'unité de commande et d'exploitation (130) est conçue pour évaluer des signaux ultrasonores reçus en ce qui concerne le temps de propagation, le décalage de fréquence, l'amplitude et/ou la position de la phase.

9. Système de thérapie et/ou de surveillance selon au moins une des revendications 1 à 8, **caractérisé en ce que** l'unité de commande et d'exploitation (130) est conçue pour détecter des décalages de fréquence de signaux ultrasonores reçus par rapport à la fréquence de signaux ultrasonores émis afin de déterminer la présence ou l'absence d'un effet Doppler.

10. Système de thérapie et/ou de surveillance selon au moins une des revendications 1 à 9, **caractérisé en ce que** l'implant (100) est un stimulateur cardiaque et/ou un moniteur cardiaque et que l'unité de commande et d'exploitation (130) est conçue pour évaluer des signaux ultrasonores reçus en tenant compte de signaux physiologiques enregistrés par l'implant (100) et représentant un cycle cardiaque respectif.

11. Système de thérapie et/ou de surveillance selon la revendication 10, **caractérisé en ce que** l'unité de commande et d'exploitation (130) est conçue pour générer un profil de mobilité de l'implant à partir de signaux ultrasonores reçus et pour évaluer un tel profil de mobilité en relation avec des signaux électro-physiologiques représentant un cycle cardiaque.

12. Système de thérapie et/ou de surveillance selon au moins une des revendications 1 à 11, **caractérisé en ce que** les signaux physiologiques enregistrés par l'implant et représentant un cycle cardiaque respectif sont des électrocardiogrammes intracardiaques.
